(19) Europäisches Patentamt
European Patent Office
Office européen des brevets

(11) EP 1 293 198 B1

(12) EUROPEAN PATENT SPECIFICATION

(45) Date of publication and mention of the grant of the patent:
**09.02.2005 Bulletin 2005/06**

(51) Int Cl.[7]: **A61K 9/70**

(21) Application number: **01121687.6**

(22) Date of filing: **14.09.2001**

# (54) Device for transdermal administration for the treatment of urinary tract disorders

Transdermal Verabreichungssystem für die Behandlung von Harnwegserkrankungen

Système d'administration transdermal pour le traitement de désordres du tract urinaire

(84) Designated Contracting States:
**AT BE CH CY DE DK ES FI FR GB GR IE IT LI LU MC NL PT SE TR**

(43) Date of publication of application:
**19.03.2003 Bulletin 2003/12**

(73) Proprietors:
- **SCHWARZ PHARMA AG**
  **D-40789 Monheim/Rhld. (DE)**
- **Kissei Pharmaceutical Co., Ltd.**
  **Matsumoto-City Nagano-Pref. 399-65 (JP)**

(72) Inventors:
- **Dressen, Frank, Dr.**
  **52391 Vettweiss (DE)**
- **Schacht, Dietrich**
  **50935 Köln (DE)**
- **Wolff, Hans-Michael, Dr.**
  **40789 Monheim (DE)**

(74) Representative: **HOFFMANN - EITLE**
**Patent- und Rechtsanwälte**
**Arabellastrasse 4**
**81925 München (DE)**

(56) References cited:
**EP-A- 0 600 675**
**EP-A- 0 799 618**
**EP-A- 0 799 619**
**WO-A-99/48530**
**WO-A-99/64042**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

FIELD OF THE INVENTION

The present invention relates to devices for the transdermal delivery of indoline compounds of formula (I). Moreover, the invention relates to the use of indoline compounds of formula (I) for the preparation of a medicament for transdermal application for treating or preventing urinary tract disorders and/or symptoms associated with these conditions.

TECHNICAL BACKGROUND

The active ingredient of the device according to the present invention is an indoline compound of formula (I)

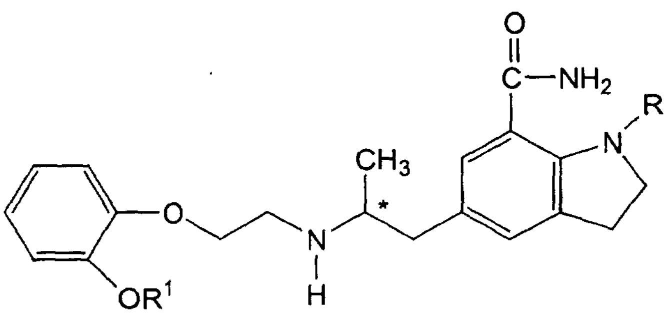

(I)

wherein
R represents a saturated or unsaturated $C_{2-7}$ aliphatic acyl group optionally substituted with one or more halogen atoms, a hydroxy group, a $C_{1-6}$ alkoxy group, a carboxyl group, a $C_{2-7}$ alkoxycarbonyl group, a 5 to 7-membered cycloalkyl group, a phenyl or naphthyl group; a $C_{2-6}$ hydroxyalkyl group; an aliphatic acyloxyalkyl group having a $C_{2-7}$ acyl group and a $C_{1-6}$ alkyl group; a $C_{1-6}$ alkyl group substituted with a $C_{1-6}$ alkoxy group, a carboxyl group, a $C_{2-7}$ alkoxycarbonyl group, a $C_{2-7}$ alkoxycarbonyl group substituted with a phenyl or naphthyl group, a carbamoyl group, a mono- or di-($C_{1-6}$ alkyl)-substituted carbamoyl group or a cyano group; a benzoyl or naphthoyl group optionally substituted with one or more halogen atoms; a furoyl group or a pyridylcarbonyl group; and
$R^1$ represents a $C_{1-6}$ alkyl group optionally substituted with one or more halogen atoms, a phenyl or a naphthyl group; the carbon atom marked "*" represents a carbon atom in (R)-configuration, (S)-configuration or a mixture thereof.

A particularly preferred compound of formula (I) is (-)-(*R*)-1-(3-hydroxypropyl)-5 [2-[[2-[2-(2,2,2-trifluoroethoxy)phenoxy]ethyl]amino]propyl]indoline-7-carboxamide (KMD 3213) which has the structure as shown below:

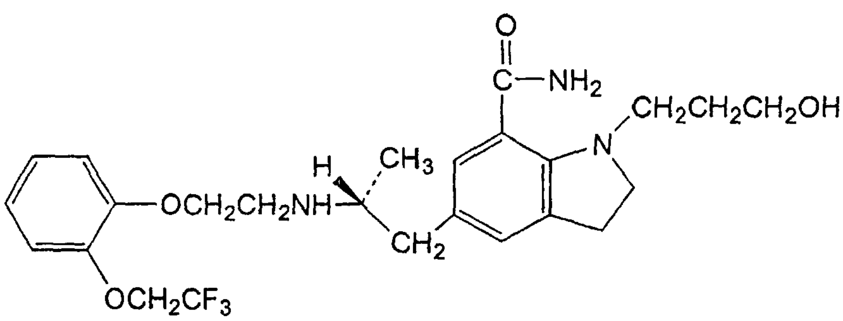

The compounds of formula (I) as well as methods for their preparation are disclosed in EP A 600 675. The compounds are said to be useful for the treatment of dysuria. KMD 3213 is disclosed in EP A 600 675 as a particularly preferred embodiment of the compounds of formula (I).

The indoline compounds of formula (I) are α-adrenoceptor antagonists. α-Adrenoceptor antagonist are generally thought to be useful in treating and preventing urinary tract disorders, such as dysuria, benign prostatic hypertrophy (BPH) or prostatic cancer (EP A 799 618 and EP A 799 619).

Furthermore, it is known that KMD 3213 is an α-adrenoceptor subtype specific antagonist, binding specifically to the $\alpha_{1a}$-receptor (S. Murata, T. Taniguchi, I. Muramatsu, British Journal of Pharmacology (1999) 127, 19-26).

Urinary outlet obstruction in patients with BPH is attributed to a mechanical component which is the urethral impression produced by the hypertrophy prostatic tissue, and a dynamic component related to the tone of urethral and prostatic smooth muscles. Stimulation of $\alpha_{1a}$-adrenoceptors of urethral and prostatic smooth muscles has been shown to cause bladder outlet obstructions in patients suffering from BPH.

Consequently, KMD 3213 was tested in animal models for its usefulness in the treatment of urinary outlet obstructions in patients with BPH (K. Akiyama, M. Hora, S. Tatemichi, N. Masuda, S. Nakamura, R. Yamagishi, M. Kitazawa, J. Pharmacol. Exp. Thera. (1999) 241, 81-91).

Moreover, it has been postulated that α-adrenoceptor antagonists are capable of preventing and treating urinary tract disorders such as BPH and prostate cancer causally (EP 799 618 and EP 799 619).

The indoline compounds of formula (I) have presently only been suggested for oral or parenteral administration. Other application forms of these compounds were not suggested previously.

In order to provide a reliable treatment for BPH the indoline compounds of formula (I), and particularly KMD 3213, have to be administered in a way that secures

(a) a sufficient bio-availability,
(b) a constant plasma level or at least a tight control of the plasma level in view of the low therapeutic index, and
(c) provides for high patient compliance.

The desired combination of (a), (b) and (c) was previously impossible to achieve as all previously used administration forms of the indoline compounds of formula (I), and in particularly KMD 3213 proved to show certain disadvantages.

Firstly, the bio-availability of those compounds of formula (I) when administered orally is very low (about 20%), and the half-life of compounds such as KMD 3213 is short, i.e. in the range of 4.5 to 10 hours.

Moreover, the relatively low therapeutic index of α-adrenoceptor antagonists such as the indoline compounds of formula (I) requires a constant plasma level without periodic concentration peaks as obtained by oral administration.

On the other hand, parenteral administration may often be undesirable for the patient in view of the higher effort and stress (e.g. fear of injections) and the higher risk involved. Furthermore, intramuscular or subcutaneous injection may cause local irritation of the skin or the underlying tissue. Finally, it is difficult to provide the necessary constant and effective plasma level even by parenteral administration unless constant infusions are applied.

The indoline compounds of formula (I) are relatively large compounds having a molecular mass of about 500 Da or more. KMD 3213 for example has a molecular mass of 495,5 Da. A person skilled in the art generally considers a molecular mass of as high as about 500 Da as being in a range where transdermal application is no longer possible, i.e. compounds having a molecular mass of about 400 Da or more are normally not suitable for transdermal administration.

Reference is made to Pfister (Pfister W.R. "Transdermal and Dermal Therapeutic Systems: Current Status" in the standard textbook of Ghosh, T.K., Pfister, W.R., Yum S.I. (eds) "Transdermal and Topical Drug Delivery Systems", Chapter 2, Interpharm Press, Buffalo Grove, Illinois 1997, pp 33-112). On page 48 thereof it is stated: "In general the desirable physicochemical, biopharmaceutical and pharmacokinetic attributes of a candidate for passive TDD include the following:... Low molecular weight, (i.e. less than 400 daltons)... "

Moreover, it is widely accepted that the MW has a large influence on the drug absorption, e.g Potts and Guy have proposed the following equation for predicting the skin permeability of drugs:

$$\log Kp\ (cm/sec) = -6{,}3 + 0.71 \log K_{oct} - 0.0061\ MW$$

(Potts, R.O. and Guy, R.H. (1992) "Predicting skin permeability". Pharmaceutical Research 9, 663-669). According to this equation, the membrane permeability constant Kp has an exponential relationship to the molecular weight (MW) of a given substance.

In view of the above, it has been very surprising that by using a device containing indoline compounds of formula (I) with a molecular size of about 500 Da, such as KMD 3213, the active agent can be administered with a good bio-availability and a steady state flux rate of about 3 mg per day for at least 72 hours resulting in uniform plasma levels of the active agent.

The plasma level obtained is sufficient to allow for a reasonable expectation that an effective prevention or treatment of urinary tract disorders such as BPH or prostatic cancer with less side effects can be provided.

It is to be understood that the term "treatment" in the context of this invention is meant to designate causal treatment or alleviation of the symptoms of urinary tract disorders.

SUMMARY OF THE INVENTION

The present invention provides a device for transdermal administration of a compound of formula (I) in order to achieve an effect in treating or preventing urinary tract disorders, such as prostatic hypertrophy (BPH), in a mammal including human. Moreover, the invention concerns the use of said compounds of formula (I) for the preparation of a medicament for transdermal administration.

BRIEF DESCRIPTION OF THE DRAWINGS

Fig. 1 Skin permeation profile on human skin for a patch formulation according to the invention

DESCRIPTION OF THE INVENTION

The present invention provides a device for transdermal administration of a compound of formula (I)

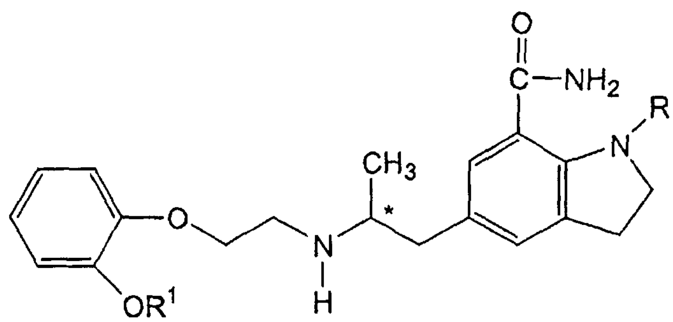

(I)

wherein
R represents a saturated or unsaturated $C_{2-7}$ aliphatic acyl group optionally substituted with one or more halogen atoms, a hydroxy group, a $C_{1-6}$ alkoxy group, a carboxyl group, a $C_{2-7}$ alkoxycarbonyl group, a 5 to 7-membered cycloalkyl group, a phenyl or naphthyl group; a $C_{2-6}$ hydroxyalkyl group; an aliphatic acyloxyalkyl group having a $C_{2-7}$ acyl group and a $C_{1-6}$ alkyl group; a $C_{1-6}$ alkyl group substituted with a $C_{1-6}$ alkoxy group, a carboxyl group, a $C_{2-7}$ alkoxycarbonyl group, a $C_{2-7}$ alkoxycarbonyl group substituted with a phenyl or naphthyl group, a carbamoyl group, a mono- or di-($C_{1-6}$ alkyl)-substituted carbamoyl group or cyano groups; a benzoyl or naphthoyl group optionally substituted with one or more halogen atoms; a furoyl group or a pyridylcarbonyl group; and
$R^1$ represents a $C_{1-6}$ alkyl group optionally substituted with one or more halogen atoms, a phenyl or a naphthyl group; the carbon atom marked "*" represents a carbon atom in (R)-configuration, (S)-configuration or a mixture thereof.

The device of the present invention is particularly useful in treating or preventing urinary tract disorders, such as benign prostatic hypertrophy (BPH) in mammals.

The term "mammals" as used herein is to be understood to mean mammalian animals including humans.

In one embodiment of the invention the device for transdermal administration may comprise a racemic mixture of compounds of formula I.

In another, more preferred embodiment of the present invention the device for transdermal administration comprises a compound of formula (I) which is essentially in its R-isomeric form or in its S-isomeric form.

The term "essentially" as used in the present invention is intended to mean at least 95%, preferably at least 97%, more preferably at least 98% and even more preferable at least 99%. In other words, a compound that is essentially in its R-isomeric form is in an amount of at least 95% in its R-isomeric form and in an amount of less than 5% in the corresponding S-isomeric form.

In a preferred embodiment of the invention, the device for transdermal administration comprises a compound of formula (I) which is (-)-(R)-1-(3-hydroxypropyl)-5-[2-[[2-[2-(2,2,2-trifluoroethoxy)phenoxy]amino]propyl]-indoline-7-carboxamide (KMD 3213).

For achieving an effect in treating or preventing urinary tract disorders the compounds of formula (I) preferably have to be administered through mammalian skin in a steady state flux rate of at least 0.5 mg per day. More preferred are steady state flux rates of at least 1mg/day, even more preferred are flux rates of 1-5 mg/day and especially preferred

are flux rates of 2-4 mg/day.

As shown in Examples 1 and 2 (see further below) with the devices provided by the present invention, steady state flux rates of KMD 3213 through human skin of 2.9 mg/day and through mouse skin of 2.7 mg/day could be achieved.

As shown in Figure 1 the steady state flux rate is maintained for at least 72 hours after the administration of the device to the patients skin. Once the typical lag phase after the initial administration of the patch has been overcome, a continuous plasma level is thus achievable.

A preferred embodiment of the present invention is therefore a device for the transdermal administration characterized in that it allows for the administration of a compound of formula (I) through human and/or animal skin in a steady state flux rate of at least 0.5 mg/day, more preferred at least 1 mg/day, even more preferred 1-5 mg/day and in particular preferred 2-4 mg/day.

The device of the present invention may be applied to the patient's skin for at least 24 hours, preferably for 48 or 72 hours or even 7 days.

In order to achieve the desired flux rate it is important to have a sufficient concentration of dissolved drug incorporated in the device. In a preferred embodiment of the present invention the device comprises at least one layer wherein the drug is dissolved in a concentration of at least 1% (w/w), preferably about 1 to 10% (w/w), most preferably 3-7% (w/w) and particularly preferred 4-6% (w/w).

Said device of the present invention comprises transdermal therapeutic systems (TTS), such as patches or systems wherein the release of the compounds is controlled by electric or osmotic means, e.g. iontophoretic or osmotic devices which are known to the skilled artisan.

In a preferred embodiment the device of the present invention is a patch.

In general, there are two types of designs for patches: a "reservoir" type and a "matrix" type.

In the reservoir type the drug, which is typically in the form of a fluid, is contained within a walled reservoir whose basal surface is permeable to the drug. The reservoir type of TTS may contain several additional layers such as a backing layer, a semi-permeable membrane which controls the drug release rate, an adhesive layer and a removable protective film.

In the matrix type the drug is dispersed in a polymer layer.

The systems of the matrix type in their simplest version comprise a one phase (monolayer) matrix. They consist of a backing layer, a self adhesive matrix containing the active agent and a protective film which is removed prior to use.

More complicated versions comprise multi-layer matrices, wherein the drug may be contained in one or more non-adhesive polymer layers.

The TTS according to the present invention is preferably a matrix system. More preferably it is a one phase (monolayer) matrix system.

Usually the adhesive will be a pressure sensitive adhesive (PSA) or a mixture of such adhesives and will form a matrix in which the active ingredient and the other components of the TTS are incorporated.

Moreover, the adhesive should preferably be pharmaceutically acceptable in a sense that it is biocompatible, non-sensitising and non-irritating to the skin. Particularly advantageous adhesives for use in the present invention should further meet the following requirements:

1. Retained adhesive and co-adhesive properties in the presence of moisture or perspiration, under normal temperature variations,

2. Good compatibility with the compounds of formula (I), such as KMD 3213, as well as with the further excipients used in the formulation.

3. Providing sufficient solubility of the compounds of formula (I), such as KMD 3213, especially of the free base form of said compounds.

Although different types of pressure sensitive adhesives may be used in the present invention, it is preferred to use adhesives showing dissolution parameters which are similar to those of the compounds of formula (I). Such a preferred pressure sensitive adhesive for use in the device of the present invention can be an adhesive of the polyacrylate type.

Polyacrylates are produced by radical polymerization of (meth)acrylic acid derivatives, wherein other suitable compounds, such as for example vinyl acetate may be used as further monomers. It is to be understood that as used herein the term "polyacrylate" comprises polymers comprising units derived from acrylic acid and/or methacrylic acid as well as copolymers and mixtures thereof.

By selection of suitable monomers, the resulting adhesives may be designed in order to have specific properties, i.e. a favourable dissolving capacity for the active agent, a desired moveability of the active agent in the matrix as well as a desired transfer rate via the skin. The transfer rate is essentially determined by the distribution coefficient and the resorption of the active agent by the skin.

The pressure sensitive adhesive of the polyacrylate type may be a homopolymer and/or copolymer of at least one acrylic and/or methacrylic acid derivative in the form of a solution in an organic solvent (solution type). The polyacrylate type adhesive may be in a crosslinkable or non-crosslinkable form. The crosslinking agent links the polymer chains via reactive groups. This may result in increased cohesion of the adhesive.

Advantageously, the polymer adhesive of the polyacrylate type contains at least one of the following monomers:

Acrylic acid, acrylamide, hexyl acrylate, 2-ethyl-hexyl acrylate, hydroxy ethyl acrylate, octyl acrylate, butyl acrylate, methyl acrylate, glycidyl acrylate, methyl acrylate, methacrylic acid, methacrylamide, hexyl methacrylate, 2-ethylhexylamide acrylate, octyl methacrylate, methyl methacrylate, glycidyl methacrylate, vinyl acetate, vinylpyrrolidone, allyl acrylate.

More preferably the polymer adhesives of the acrylate type are cross-linkable adhesives polymerized from a combination of the following monomers:

2-ethyl-hexyl acrylate/n-butyl acrylate/butyl acrylate/acrylic acid,
2-ethyl-hexyl acrylate/n-butyl acrylate/vinyl acetate/acrylic acid,
2-ethyl-hexylacrylate/vinyl acetate/acrylic acid,
2-ethyl-hexyl acrylate/vinyl acetate/allyl acrylate,
2-ethyl-hexyl acrylate/vinyl acetate/divinyl benzene/acrylic acid,
2-ethyl-hexyl acrylate/vinyl acetate/allyl methacrylate/acrylic acid,
2-ethyl-hexyl acrylate/vinyl acetate/2-hydroxy-ethyl acrylate,
2-ethyl-hexyl acrylate/ vinyl acetate/2-hydroxy-ethyl methacrylate,
2-ethyl-hexyl acrylate/fumaric acid diethyl ester/acrylic acid,
2-ethyl-hexyl acrylate/maleic acid diethyl ester/2-hydroxy-ethyl acrylate.

As the preferred crosslinking agents the following compounds may be mentioned: diphenyl-methan-4-diisocyanate, hexamethylene diisocyanate, isophoron diisocyanate, titanium acetylacetonate, aluminium acetylacetonate, iron acetylacetonate, zinc acetylacetonate, magnesium acetylacetonate, zirconium acetylacetonate, 2-ethyl-1,3-hexanediol-titanate, tetra-iso-octyl-titanate, tetra-nonyl-titanate, polyfunctional propylene imine derivatives, ether derivatives of melamine formaldehyde resins, highly methylated urethane resins, imino melamine resins.

The non-crosslinkable adhesives of the solution type may advantageously be polymerized from a combination of the following monomers:

2-ethyl-hexyl acrylate/n-butyl acrylate/vinyl acetate,
2-ethyl-hexyl acrylate/vinyl acetate,
2-ethyl-hexyl acrylate/n-butyl acrylate/vinyl acetate/allyl acrylate,
2-ethyl-hexyl acrylate /n n-butyl acrylate/allyl methacrylate,
2-ethyl-hexyl acrylate/n-butyl acrylate/vinyl acetate/divinyl benzene,
2-ethyl-hexyl acrylate/fumaric acid diethyl ester/allyl acrylate,
2-ethyl-hexyl acrylate/maleic acid diethyl ester/allyl acrylate,
2-ethyl-hexyl acrylate/n-butyl acrylate/acrylamide/vinyl acetate/allyl acrylate,
2-ethyl-hexyl acrylate/n-butyl acrylate/iso-butyl acrylate/vinyl acetate/allyl acrylate.

Moreover, some adhesives may be used in the form of aqueous dispersions (dispersion type). The use of these dispersion type adhesives may involve the further advantage that during coating and drying no flammable or toxic solvents evaporate.

Dispersion type adhesives may advantageously be polymerized from a combination of the following monomers:

n-butyl acrylate/iso-butyl acrylate/acrylic acid
2-ethyl-hexyl acrylate/n-butyl acrylate/acrylic acid,
2-ethyl-hexyl acrylate/n-butyl acrylate/2-hydroxy-ethyl acrylamide,
2-ethyl-hexyl acrylate/n-butyl acrylate/vinyl acetate/acrylamide,
2-ethyl-hexyl acrylate/n-butyl acrylate/vinyl acetate/ 2-hydroxy-ethyl acrylate,

2-ethyl-hexyl acrylate/n-butyl acrylate/allyl acrylate/acrylic acid,
2-ethyl-hexyl acrylate/n-butyl acrylate/vinyl acetate/divinyl benzene.

Preferred polyacrylates for use in the present invention are cross-linked using multivalent metal ions, in order to improve the physical properties of the adhesive or to adapt it to the specific requirements. The metal ions are normally employed in the form of metal chelates which are soluble in organic solvents. Particularly suitable crosslinking agents are aluminium acetyl acetonate and titanium acetyl acetonate.

If the adhesive used according to the present invention is a polyacrylate adhesive, the solvent capacity is generally dependent on the type and amount of free functional groups in the adhesive.

Most preferred adhesives for use in the device of the present invention are polyacrylates with polar groups, especially with free hydroxy- and/or carboxyl groups. Examples of such adhesives are polyacrylates wherein polar monomers such as e.g. hydroxy-ethyl acrylate, hydroxy-ethyl methacrylate, acrylic acid or methacrylic acid in an amount of about 1-10% (w/w), more preferred in an amount of 3-8% (w/w), most preferred in an amount of 4-6% (w/w) are used. Those adhesives are commercially available under the tradename Duro-Tak® (National Starch & Chemicals; Hamburg).

Even more preferred for use in the device of the present invention are adhesives of the polyacrylate type wherein hydroxy-ethyl acrylate and/or hydroxy-ethyl methacrylate monomers are incorporated during polymerization in an amount of 3-8% (w/w), most preferred in an amount of 4-6% (w/w).

Such an adhesive may be obtained in line with the general procedure described in US Patent 5,498,418 as follows: The adhesive can be obtained by radical polymerisation in a first stage of a mixture consisting of 21 to 40% by weight vinyl acetate, 55 to 70% by weight of an acrylic acid-$C_{2-8}$-alkyl ester, and 3 to 10% by weight of an acrylic acid-$C_{2-4}$-hydroxyl acryl ester, with 100% by weight monomers in the mixture, in an organic solvent, whereafter in a second stage a conventional crosslinking agent in an organic solvent and the active ingredient in the quantity required for the intended use of the transdermal device (plaster) is admixed, if necessary in an organic solvent, and finally in a third stage the resulting mixture of the particular acrylate-vinyl acetate copolymer is crosslinked in an additional stage, accompanied by heating and the removal of the organic solvent or mixture of solvents used; the resulting active ingredient is "built into" the adhesive substance in a special manner by the subsequent and additional crosslinkage of the special acrylate-vinyl acetate copolymer. The acrylate-vinyl acetate copolymer has a relative viscosity of 3.0 to 4.2 at 20°C.

Preferably the mixture of monomers contains 2-ethylhexylacrylate and hydroxyethylacrylate in addition to vinyl acetate. Preferably the subsequent crosslinkage of the special acrylate-vinyl acetate copolymer is performed with a titanium acid ester consisting of polybutyl titanate and/or titanium acetylacetonate, more particularly in a quantity of 0.3 to 3% by weight thereof, the percentages by weight being related to the weight of the copolymer.

A process for producing a TTS according to the invention may also include the steps of applying a solution of a copolymer, containing the active ingredient in the required amount for the intended use of the TTS and a conventional crosslinker or mixture thereof, and obtained by the radical polymerisation of a mixture of monomers consisting of 21 to 40 % by weight vinyl acetate, 55 to 70% by weight of an acrylic acid-$C_{2-8}$-alkyl ester and 1 to 10% by weight of an acrylic acid-$C_{2-4}$-hydroxyalkylester, in the required layer thickness to the protective film of the TTS, and removing the solvent or mixture of solvents by heating, thus effecting an additional crosslinking of the special acrylate-vinyl acetate copolymer.

One embodiment of such a process is characterized in that the acrylate-vinyl acetate copolymer, the active ingredient and the crosslinking agent are initially dissolved in a solvent which contains 20 to 40% by weight of ethanol or an ethanol-methanol mixture, with a solids proportion consisting of 40 to 60% by weight of the mixture of the special acrylate-vinyl acetate copolymer, crosslinking agent and the active ingredient.

A particular working example for the preparation of such a acrylate-vinyl acetate adhesive is disclosed in US-A-5,498,418, col.2, line 61 to col. 3, line 10.

An especially preferred adhesive for use in the present invention is the commercially available adhesive DuroTak® 387-2287 (National Starch & Chemicals; Hamburg).

For the preparation of the TTS according to the invention, a compound of formula (I), such as KMD 3213, is solved or suspended in ethanol or another suitable organic solvent and subsequently the adhesive is added while stirring.

If the adhesive contains a suitable solvent system, the active agent may be directly added to the adhesive solution. Further auxiliary agents may be added either to the adhesive solution, the solution of the active agent or the adhesive solution containing the active agent.

In one particular preferred embodiment of the present invention the device of the present invention is a one phase matrix, wherein one or more compounds of formula (I) are dissolved in a pressure sensitive adhesive of the acrylate type in an amount of 1-10% (w/w), preferably 3-7% (w/w), and most preferred 4-6% (w/w), and wherein said acrylate preferably contains free polar groups, such as hydroxy groups or carboxy groups, as described further above.

The thickness of the adhesive layer may be between 0.01 and 0.30 mm, preferably between 0.02 and 0.20 mm and most preferably between 0.03 and 0.10 mm.

In a further preferred embodiment, the device further includes a solubilizer.

A solubilizer is an additive that inhibits the crystallisation of the active agent during storage of the device. Examples of solubilizers known in the art are, e.g. phthalic acid esters, adipic acid ester, monoglycerides, diglycerides, triglycerides, fatty acids and esters and derivatives thereof, higher alcohols and their derivatives, derivatives of nonylphenol or octylphenol, derivatives of sorbitol or mannitol, non-ionogenic tensides, polyoxyethylene alkyl ethers, derivatives of ricinus oil, sitosterin or polyvinylpyrrolidone.

Preferred solubilizers for use in the device of the present invention are solubilizers which are capable of keeping the compounds of formula (I) in solution if said device is stored for a prolonged period of time.

It is presently considered that preferred solubilizers for inhibiting the precipitation of the compounds of formula (I) are additives having a pKa of about 4.5 to 6.0, more preferred 4.5 to 5.5, even more preferred between 4.8 and 5.1 and particularly preferred about 5.0 such as e.g. carboxylic acids, including particularly fatty acids.

In a preferred embodiment the devices of the present invention thus contain a solubilizer, wherein said solubilizers are carboxylic acids. Most preferred solubilizers are fatty acids having a $pK_a$ of 4.8 to 5.1 and at least 10 carbon atoms and which may or may not have one or more double bonds.

In an even more preferred embodiment of the present invention the solubilizer may be chosen from the group comprising lauric acid, linoleic acid, stearic acid, palmitic acid and oleic acid.

A particularly preferred solubilizer for use in the device of the present invention is oleic acid.

The solubilizer is preferably present in an amount of about 50 to 500 mol%, more preferred in an amount of 100 to 400 mol%, even more preferred in an amount of 100 to 300 mol% and particularly preferred in an amount of about 200 mol% based on the active compound.

If for example KMD 3213 is used as the active ingredient, then oleic acid is preferably used in an amount of approximately 50 to 500 mol %, i.e. in an amount of 28 to 280% (w/w) based on the amount of KMD 3213.

The device of the present invention may therefore contain oleic acid in a concentration of about 0,28% to 28% (w/w) in a layer containing 1 to 10% (w/w) KMD 3213. Typically, KMD 3230 may be present in a concentration of 5% (w/w) in the adhesive layer, which may also contain about 5 to 6% (w/w) oleic acid.

The device of the present invention may further comprise a penetration enhancer. Penetration enhancers are additives which enhance the penetration of the active agent through mammalian skin. Examples of penetration enhancers are well known to the skilled artisan and comprise citrate; fatty acids; fatty acid esters; glycerol and esters thereof such as e.g. glycerol monolaurate (GML); alcohols with up to 8 carboxylic atoms, such as ethanol, 1,2-propandiol, dexpanthenol or polyethyleneglycol; mixtures of alcohol and water; vitamin E and derivatives thereof; copolymers of ethylene and vinyl acetate; polyvinylpyrrolidone; copolymers of polyvinylpyrrolidone and vinyl acetate; polypropyleneglycol.

Although the addition of a penetration enhancer may be possibly useful in some embodiments of the invention, it is usually not necessary. As shown in Example (I), flux rates of 2.7 to 2.9 mg KMD 3213 per day were achieved using a one phase matrix type TTS.

In a preferred embodiment the device of the present invention is therefore devoid of any additive that enhances the penetration of the compounds of formula (I) through mammalian skin.

In other embodiments of the invention the device may comprise additional additives such as stabilizers or swellable agents which are well known to the skilled artisan.

In a preferred embodiment of the present invention, the device has a basal surface area of 5 to 50$cm^2$, particularly of 10 to 20$cm^2$. It goes without saying that a device having a surface area of, say, 20$cm^2$ is pharmacologically equivalent to and may be exchanged by two 10$cm^2$ devices or four 5$cm^2$ devices having the same drug content per $cm^2$. Thus, the surface areas as indicated herein should be understood to refer to the total surface of all devices simultaneously administered to a patient.

Providing and applying one or several devices according to the invention has the pharmacological advantage over oral therapy that the attending physician can titrate the optimum dose for the individual patient relatively quickly and accurately, e.g. by simply increasing the number or size of devices given to the patient. Thus, the optimum individual dosage can often be determined after a time period of only about 3 weeks with low side effects.

A preferred content of a compound of formula I, such as KMD 3213 in the devices according to the invention is in the range of 0.1 to 2.0 mg/$cm^2$. Still more preferred are 0.20 to 1.0 mg/$cm^2$. If a 7 day patch is desired, higher drug contents will generally be required.

The device used in the present invention is preferably a patch having a continuous adhesive matrix in at least its center portion containing the drug. However, transdermal equivalents to such patches are likewise comprised by the present invention, e.g. an embodiment where the drug is in an inert but non-adhesive matrix in the center portion of the device and is surrounded by an adhesive portion along the edges.

The device of the present invention may further comprise a backing film, which is a film being impermeable

to the active compounds. Such a film may consist of polyester, polyamide, polyethylene, polypropylene, polyurethane, polyvinylchloride or of combinations of the aforementioned materials which may or may not be coated with an aluminium film or with aluminium vapour. The thickness of the backing film may be between 10 and 100 µm, preferably between 20 and 40 µm.

The device of the present invention may further contain a release liner foil, which will be removed immediately prior before the device will be brought into contact with the mammalian skin. The release liner foil may consist of polyester, polyethylene or polypropylene which may or may not be coated with aluminium film or aluminium vapour or fluoropolymers. Typically, the thickness of such a release liner ranges between 20 and 300 µm and preferably between 50 and 100 µm.

The device according to the present invention is prepared by a manufacturing process comprising preparing a drug loaded adhesive, coating, drying or cooling and lamination to get the bulk product, converting the laminate into patch units via cutting and packaging.

In a further aspect, this invention relates to the use of a compound of formula (I), for example KMD 3213, for preparing a medicament for transdermal administration for treating or preventing urinary tract disorders, such as benign prostate hypertrophy and prostatic cancer, and/or symptoms associated with these conditions.

Such a medicament may comprise the devices described above as well as ointments, creams, sprays, gels or films, and the like, provided that a steady state flux rate of at least 0.5 mg per day through the mammalian skin is achieved. Even more preferred are steady state flux rates of at least 1 mg/day, e.g. 1 to 5 mg/day or 2 to 4 mg/day.

In a preferred embodiment a compound of formula (I) is used for preparing a medicament, wherein the medicament is a patch of the matrix type, and most preferably a patch of the one phase matrix type.

In a particular preferred embodiment a compound of formula (I) is used for preparing a medicament, which is a one phase matrix type patch, wherein said compound is dissolved in the adhesive layer and wherein the adhesive is a of the polyacrylate type including polar groups.

The invention and the best mode for carrying it out will be explained in more detail in the following examples.

Example 1

A TTS using a polyacrylate based pressure sensitive adhesive was prepared as follows.

1g of (-)-(*R*)-1-(3-hydroxypropyl)-5-[2-[[2-[2-(2,2,2-trifluoroethoxy)phenoxy]ethyl]amino]propyl]indoline-7-carboxamide (KMD 3213) was solved in 8g ethanol, 1g of oleic acid and 32,1g of a solution containing 18 g of DuroTak® 387-2287 (in ethylacetate) were added while stirring. The resulting mixture was stirred (350 u/min) approximately one hour until a homogenous dispersion was obtained.

The dispersion was coated onto a polyester release liner (SCOTCHPAK® 1022) with a suitable doctor knife and the solvents were removed in a drying oven at a temperature of 50°C for about 30 minutes to obtain an adhesive matrix of 76g/m$^2$ coating weight, which contained 5% (w/w) drug. The dried matrix film was laminated with a polyester type backing foil (SCOTCHPAK® 1109). The individual patches were punched out of the complete laminate to a desired patch size (for example 5cm$^2$, 10cm$^2$, 20cm$^2$, 30cm$^2$) and sealed into pouches under the flow of nitrogen. The obtained patches were studied using several test methods.

Example 2

A TTS using a polyacrylate based pressure sensitive adhesive was prepared as follows.

1g of KMD 3213 was solved in 8g ethanol, 1,2 g of oleic acid and 31.8g of a solution containing 17,8g of DuroTak® 387-2287 (in ethylacetate) were added while stirring. The resulting mixture was stirred (350 u/min) approximately one hour until a homogenous dispersion was obtained.

The dispersion was coated onto a polyester release liner (SCOTCHPAK® 1022) with a suitable doctor knife and the solvents were removed in a drying oven at a temperature of 50°C for about 30 minutes to obtain an adhesive matrix of 81g/m$^2$ coating weight, which contained 5% (w/w) drug. The dried matrix film was laminated with a polyester type tacking foil (SCOTCHPAK® 1109). The individual patches were punched out of the complete laminate to a desired patch size (for example 5cm$^2$, 10cm$^2$, 20cm$^2$, 30cm$^2$) and sealed into pouches under the flow of nitrogen. The obtained patches were studied using several test methods.

Example 3

0,75g of KMD 3213 was solved in 4g ethanol, 0,5 g of Kollidon 90F and 15,6g of a solution containing 8,75g DuroTak® 387-2287 (in ethylacetate) were added while stirring. The resulting mixture was stirred (700 u/min) approximately one hour until a homogenous dispersion was obtained.

The dispersion was coated onto a polyester release liner (SCOTCHPAK® 1022) with a suitable doctor knife

and the solvents were removed in a drying oven at a temperature of 50°C for about 30 minutes to obtain an adhesive matrix of 90g/m$^2$ coating weight, which contained 7,5% (w/w) drug. The dried matrix film was laminated with a polyester type tacking foil (SCOTCHPAK® 1109). The individual patches were punched out of the complete laminate to a desired patch size (for example 5cm$^2$, 10cm$^2$, 20cm$^2$, 30cm$^2$) and sealed into pouches under the flow of nitrogen. The obtained patches were studied using several test methods.

Example 4

0.5g of KMD 3213 was solved in 4g ethanol, 0.5 g of isopropylmyristate and 16,1g of a solution containing 9g DuroTak® 387-2287 (in ethylacetate) were added while stirring. The resulting mixture was stirred (700 u/min) approximately one hour until a homogenous dispersion was obtained.

The dispersion was coated onto a polyester release liner (SCOTCHPAK® 1022) with a suitable doctor knife and the solvents were removed in a drying oven at a temperature of 50°C for about 30 minutes to obtain an adhesive matrix of 55g/m$^2$ coating weight, which contained 5% (w/w) drug. The dried matrix film was laminated with a polyester type tacking foil (SCOTCHPAK® 1109). The individual patches were punched out of the complete laminate to a desired patch size (for example 5cm$^{2,}$ 10cm$^2$, 20cm$^2$, 30cm$^2$) and sealed into pouches under the flow of nitrogen. The obtained patches were studied using several test methods.

**Drug release in vitro (flux across dialysis membrane):**

The assay was performed according to Tanojo et al. (Journal of Controlled Release 45 (1997), 41-47).

In this test, the patch showed a high release rate per area unit. Due to a fast depletion of the reservoir the drug release rate was time dependent as expected.

**Skin Permeation:**

The skin permeation of KMD 3213 was also evaluated on hairless mouse skin and human skin. Human skin penetration assays were performed as described by Tanojo et al. in Journal of Controlled Release 45 (1997), 41-47. The concentration of KMD 3213 was measured by HPLC with a Symetry Shield RP-8, 3.9*150 mm 5 µm column; T=35°C; eluent: water/acetonitrile/trifluoracetic acid 800/200/1 (v/v/v) ; dectetion UV at 225 nm; flux rate 1.5 ml/min; injection volume: 100 µl at 23°C.

The flux measurements on hairless mouse skin were conducted using a TTS area of 2.55 cm$^2$ fixed on mouse abdominal and back skin in a horizontal diffusion cell. Immediately thereafter the acceptor chamber of the cell was filled with phosphate buffer solution (0.066 molar) previously adjusted to 32 ± 0.5°C and pH 6.2, in an air-bubble free state, and the release medium thermostatted to 32 ± 0.5°C. At the time of sampling (after 3, 6, 24 and 48 hours) the release medium is exchanged with fresh medium thermostatted at 32 ± 0.5 °C. KMD 3213 was measured with HPLC as explained above.

On hairless mouse skin a steady state skin permeation rate of approximately 2.7mg/20cm$^2$ per day was observed. On human skin the steady state flux rate was equivalent to 2.9mg/20cm$^2$ per day.

The cumulative permeation rate on human skin of the device produced according to example 1 is shown in Fig. 1.

The permeation rates on human or mouse skin of the devices produced according to examples 1 to 4 are shown in table 1.

Table 1:

| Penetration of KMD 3213 (µg/5cm$^2$) through human skin (Examples 1 and 2) and through mouse skin (Examples 3 and 4) | | | | |
|---|---|---|---|---|
| | 6 hours | 24 hours | 48 hours | 72 hours |
| Example 1 | 5,1 | 20,1 | 206,1 | 832,35 |
| Example 2 | 1,8 | 23,25 | 335,25 | 1030,8 |
| Example 3 | 97,3 | 735,5 | 1152,1 | n.d. |
| Example 4 | 81 | 599,2 | 1000,8 | n.d. |
| n.d.=not determined | | | | |

**Stability:**

Stability tests of the devices produced according to Examples 1 and 2 revealed that nucleation or crystal growth could not be observed during a storage at 40°C and 75% relative humidity for at least 3 months.

## Claims

1. A device for transdermal administration, containing a compound of the formula (I)

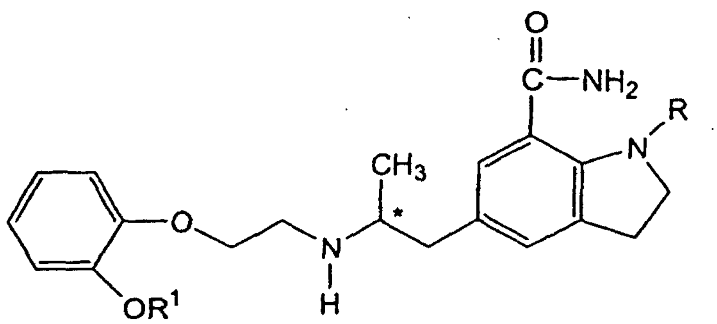

(I)

wherein
R represents a saturated or unsaturated $C_{2-7}$ aliphatic acyl group optionally substituted with one or more halogen atoms, a hydroxy group, a $C_{1-6}$ alkoxy group, a carboxyl group, a $C_{2-7}$ alkoxycarbonyl group, a 5 to 7-membered cycloalkyl group, a phenyl or naphthyl group; a $C_{2-6}$ hydroxyalkyl group; an aliphatic acyloxyalkyl group having a $C_{2-7}$ acyl group and a $C_{1-6}$ alkyl group; a $C_{1-6}$ alkyl group substituted with a $C_{1-6}$ alkoxy group, a carboxyl group, a $C_{2-7}$ alkoxycarbonyl group, a $C_{2-7}$ alkoxycarbonyl group substituted with a phenyl or naphthyl group, a carbamoyl group, a mono- or di-($C_{1-6}$ alkyl)-substituted carbamoyl group or a cyano group; a benzoyl or naphthoyl group optionally substituted with one or more halogen atoms; a furoyl group or a pyridylcarbonyl group; and
$R^1$ represents a $C_{1-6}$ alkyl group optionally substituted with one or more halogen atoms, a phenyl or a naphthyl group;
the carbon atom marked "*" represents a carbon atom in (R)-configuration, (S)-configuration or a mixture thereof.

2. The device for transdermal administration according to claim 1, **characterized in that** said compound of formula (I) essentially is in its R-isomeric form, S-isomeric form or in its racemic form.

3. The device for transdermal administration according to any one of the preceding claims, **characterized in that** said compound of formula (I) is (-)-(R)-1-(3-hydroxypropyl)-5-[2-[[2-[2-(2,2,2-trifluoroethoxy)phenoxy]ethyl]amino] propyl]-indoline-7-carboxamide (KMD 3213).

4. The device according to any one of the preceding claims, **characterized in that** it administers a compound of formula (I) through mammalian skin in a steady state flux rate of at least 0.5 mg per day.

5. The device for transdermal administration according to any one of the preceding claims, **characterized by** having a loading of a compound represented by formula (I) from about 0.1 - 2 $mg/cm^2$.

6. The device according to any one of the preceding claims, **characterized in that** it comprises at least one layer wherein a compound of formula (I) is dissolved in a concentration of at least 1 % (w/w).

7. The device according to claim 6, wherein the compound of formula (I) is dissolved in a concentration between 3 % and 7 % (w/w).

8. The device according to any one of the preceding claims, **characterized in that** it is a patch of the reservoir or the matrix type.

**9.** The device according to any one of the preceding claims, **characterized in that** it is a patch of the matrix type, wherein said compound of formula (I) is dissolved in the adhesive.

**10.** The device according to any one of the preceding claims, **characterized in that** it contains an adhesive of the polyacrylate type.

**11.** The device according to any one of the preceding claims, **characterized in that** it further comprises a solubilizer.

**12.** The device according to claim 11, wherein the solubilizer is a carboxylic acid.

**13.** The device according to claims 11 or 12, wherein the solubilizer is oleic acid.

**14.** The device according to any one of claims 11 to 13, wherein the solubilizer is present in an amount of 50 to 500 mol% based on the amount of the compound of formula (I) that is incorporated in the device.

**15.** The device according to any of the preceding claims, wherein the device has a basal area of 5 to 50 $cm^2$.

**16.** The device according to any one of the preceding claims, **characterized in that** it releases the compound represented by formula (I) for a predefined period of time, preferably for 48 or 72 hours, or up to 7 days.

**17.** The use of a compound of formula (I)

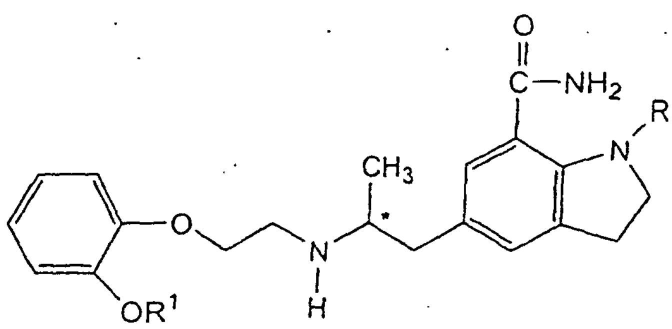

(I)

wherein
R represents a saturated or unsaturated $C_{2-7}$ aliphatic acyl group optionally substituted with one or more halogen atoms, a hydroxy group, a $C_{1-6}$ alkoxy group, a carboxyl group, a $C_{2-7}$ alkoxycarbonyl group, a 5 to 7-membered cycloalkyl group, a phenyl or naphthyl group; a $C_{2-6}$ hydroxyalkyl group; an aliphatic acyloxyalkyl group having a $C_{2-7}$ acyl group and a $C_{1-6}$ alkyl group; a $C_{1-6}$ alkyl group substituted with a $C_{1-6}$ alkoxy group, a carboxyl group, a $C_{2-7}$ alkoxycarbonyl group, a $C_{2-7}$ alkoxycarbonyl group substituted with a phenyl or naphthyl group, a carbamoyl group, a mono- or di-($C_{1-6}$ alkyl)-substituted carbamoyl group or a cyano group; a benzoyl or naphthoyl group optionally substituted with one or more halogen atoms; a furoyl group or a pyridylcarbonyl group; and
$R^1$ represents a $C_{1-6}$ alkyl group optionally substituted with one or more halogen atoms, a phenyl or a naphthyl group;
the carbon atom marked "*" represents a carbon atom in (R)-configuration, (S)-configuration or a mixture thereof for the preparation of a medicament for transdermal application for treating or preventing an urinary tract disorder and/or symptoms associated with this condition.

**18.** The use according to claim 17, wherein the urinary tract disorder is benign prostatic hypertrophy.

**19.** The use according to anyone of claims 17 - 18, **characterized in that** said compound formula (I) essentially is in its R-isomeric form, its S-isomeric form or its racemic form.

**20.** The use according to anyone of claims 17 - 18, wherein the compound of formula (I) is (-) -(R)-1-(3-hydroxypropyl)-5-(2-[[2-[2-(2,2,2-trifluoroethoxy)phenoxy] ethyl] amino]propyl]-indoline-7-carboxamide (KMD 3213).

**21.** The use according to anyone of claims 17 - 20, wherein said compound of formula (I) is administered through mammalian skin in a steady state flux rate of at least 0.5 mg/day.

**22.** The use according to anyone of claims 17 - 21, wherein the medicament for transdermal application is the device for transdermal administration according to anyone of claims 1 - 16.

**23.** The use according to anyone of claims 17 - 21, wherein the medicament for transdermal application is an ointment, cream, spray, gel or film.

**Patentansprüche**

**1.** Eine Vorrichtung zur transdermalen Verabreichung enthaltend eine Verbindung der Formel (I),

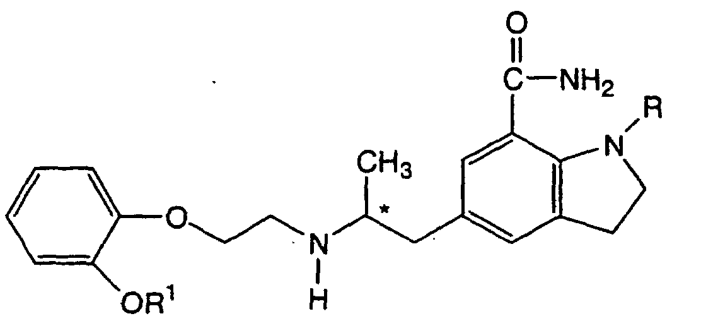

(I)

in der bedeuten:

R eine gesättigte oder ungesättigte $C_{2-7}$ aliphatische Acylgruppe, gegebenenfalls substituiert mit einem oder mehreren Halogen-Atomen, einer Hydroxy-Gruppe, einer $C_{1-6}$ Alkoxy-Gruppe, einer Carboxyl-Gruppe, einer $C_{2-7}$ Alkoxycarbonyl-Gruppe, einer 5 bis 7-gliedrigen Cycloalkyl-Gruppe, einer Phenyl- or Naphthyl-Gruppe; eine $C_{2-6}$ Hydroxyalkyl-Gruppe; eine aliphatische Acyloxyalkyl-Gruppe, die eine $C_{2-7}$ Acyl-Gruppe und eine $C_{1-6}$ Alkyl-Gruppe trägt; eine $C_{1-6}$ Alkyl-Gruppe, substituiert mit einer $C_{1-6}$ Alkoxy-Gruppe, einer Carboxyl-Gruppe, einer $C_{2-7}$ Alkoxycarbonyl-Gruppe, einer $C_{2-7}$ Alkoxycarbonyl-Gruppe, substituiert mit einer Phenyl- oder Naphthyl-Gruppe, einer Carbamoyl-Gruppe, einer Mono- oder Di-($C_{1-6}$ Alkyl)-substituierten Carbamoyl-Gruppe oder einer Cyano-Gruppe; eine Benzoyl- oder Naphthoyl-Gruppe, gegebenenfalls substituiert mit einem oder mehreren Halogen-Atomen; eine Furoyl-Gruppe oder eine Pyridylcarbonyl-Gruppe; und

$R^1$ eine $C_{1-6}$ Alkyl-Gruppe, gegebenenfalls substituiert mit einem oder mehreren Halogen-Atomen, einer Phenyl- oder einer Naphthyl-Gruppe;

das durch "*" gekennzeichnete Kohlenstoff-Atom repräsentiert ein Kohlenstoff-Atom in (R)-Konfiguration, (S)-Konfiguration oder eine Mischung daraus.

**2.** Die Vorrichtung zur transdermalen Verabreichung nach Anspruch 1, **dadurch gekennzeichnet, dass** die besagte Verbindung der Formel (I) essentiell in ihrer R-isomerischen Form, S-isomerischen Form oder in ihrer razemischen Form vorliegt.

**3.** Die Vorrichtung zur transdermalen Verabreichung nach einem der vorhergehenden Ansprüche, **dadurch gekennzeichnet, dass** die besagte Verbindung der Formel (I) (-)-(R)-1-(3-Hydroxypropyl)-5-[2-[[2-[2-(2,2,2-Trifluoroethoxy)Phenoxy]Ethyl]Amino]Propyl]-Indolin-7-Carboxamid (KMD 3213) ist.

**4.** Die Vorrichtung nach einem der vorangehenden Ansprüche, **dadurch gekennzeichnet, dass** sie eine Verbindung der Formel (I) durch Säugetierhaut in einer gleichmäßigen Fluxrate von mindestens 0,5 mg pro Tag verabreicht.

**5.** Die Vorrichtung zur transdermalen Verabreichung nach einem der vorangehenden Ansprüche, **dadurch gekenn-**

**zeichnet, dass** sie eine Beladung mit einer Verbindung gemäß der Formel (I) von ca. 0,1 - 2 mg/$cm^2$ aufweist.

6. Die Vorrichtung nach einem der vorangehenden Ansprüche, **dadurch gekennzeichnet, dass** sie mindestens eine Schicht enthält, worin eine Verbindung der Formel (I) in einer Konzentration von mindestens 1 % (w/w) gelöst ist.

7. Die Vorrichtung nach Anspruch 6, wobei die Verbindung der Formel (I) in einer Konzentration zwischen 3 % und 7 % (w/w) gelöst ist.

8. Die Vorrichtung nach einem der vorangehenden Ansprüche, **dadurch gekennzeichnet, dass** sie ein Pflaster in Reservoir- oder in Matrix-Form ist.

9. Die Vorrichtung nach einem der vorangehenden Ansprüche, **dadurch gekennzeichnet, dass** sie ein Pflaster vom Matrix-Typ ist, in dem besagte Verbindung der Formel (I) im Kleber gelöst ist.

10. Die Vorrichtung nach einem der vorangehenden Ansprüche, **dadurch gekennzeichnet, dass** sie einen Kleber vom Polyacrylat-Typ enthält.

11. Die Vorrichtung nach einem der vorangehenden Ansprüche, **dadurch gekennzeichnet, dass** sie außerdem einen Lösungsvermittler enthält.

12. Die Vorrichtung nach Anspruch 11, wobei der Lösungsvermittler eine Carbonsäure ist.

13. Die Vorrichtung nach Ansprüchen 11 oder 12, wobei der Lösungsvermittler Ölsäure ist.

14. Die Vorrichtung nach einem der Ansprüche 11 bis 13, wobei der Lösungsvermittler in einer Menge von 50 bis 500 mol% vorkommt, basierend auf der Menge der Verbindung der Formel (I), die in die Vorrichtung eingebracht wird.

15. Die Vorrichtung nach einem der vorangehenden Ansprüche, wobei die Vorrichtung eine Basisfläche von 5 bis 50 $cm^2$ hat.

16. Die Vorrichtung nach einem der vorangehenden Ansprüche, **dadurch gekennzeichnet, dass** sie die Verbindung gemäß der Formel (I) für eine vorbestimmte Zeitspanne, vorzugsweise für 48 oder 72 Stunden, oder bis zu 7 Tagen freisetzt.

17. Die Verwendung einer Verbindung der Formel (I)

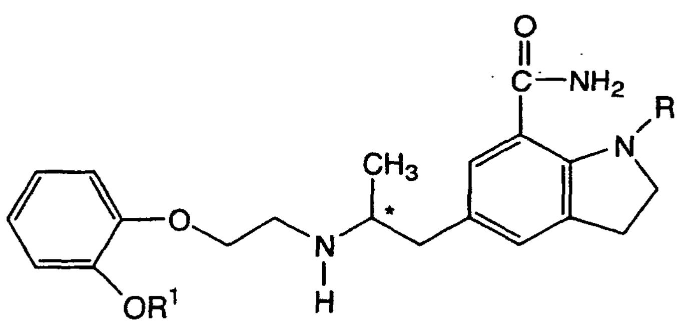

(I)

worin bedeuten:

R eine gesättigte oder ungesättigte $C_{2-7}$ aliphatische Acyl-Gruppe, gegebenenfalls substituiert mit einem oder mehreren Halogen-Atomen, einer Hydroxy-Gruppe, einer $C_{1-6}$ Alkoxy-Gruppe, einer Carboxyl-Gruppe, einer $C_{2-7}$ Alkoxycarbonyl-Gruppe, einer 5 bis 7-gliedrigen Cycloalkyl-Gruppe, einer Phenyl- oder Naphthyl-Gruppe; eine $C_{2-6}$ Hydroxyalkyl-Gruppe; eine aliphatische Acyloxyalkyl-Gruppe, die eine $C_{2-7}$ Acyl-Gruppe und eine $C_{1-6}$ Alkyl-Gruppe trägt; eine $C_{1-6}$ Alkyl-Gruppe, substituiert mit einer $C_{1-6}$ Alkoxy-Gruppe, einer Carboxyl-Gruppe, einer $C_{2-7}$ Alkoxycarbonyl-Gruppe, einer $C_{2-7}$ Alkoxycarbonyl-Gruppe, substituiert mit einer Phenyl-

oder Naphthyl-Gruppe, einer Carbamoyl-Gruppe, einer Mono- oder Di-($C_{1-6}$ Alkyl)-substituierten Carbamoyl-Gruppe oder einer Cyano-Gruppe; eine Benzoyl- oder Naphthoyl-Gruppe, gegebenenfalls substituiert mit einem oder mehreren Halogen-Atomen; eine Furoyl-Gruppe oder eine Pyridylcarbonyl-Gruppe; und

$R^1$ eine $C_{1-6}$ Alkyl-Gruppe, gegebenenfalls substituiert mit einem oder mehreren Halogen-Atomen, einer Phenyl- oder einer Naphthyl-Gruppe;

das durch "*" gekennzeichnete Kohlenstoff-Atom repräsentiert ein Kohlenstoff-Atom in (R)-Konfiguration, (S)-Konfiguration oder eine Mischung daraus zur Herstellung eines Medikaments zur transdermalen Anwendung zur Behandlung oder Vorbeugung einer Harnwegserkrankung und/oder von Symptomen, die mit diesem Zustand verbunden sind.

**18.** Die Verwendung nach Anspruch 17, wobei die Harnwegserkrankung gutartige Prostata-Hypertrophie ist.

**19.** Die Verwendung nach einem der Ansprüche 17 - 18, **dadurch gekennzeichnet, dass** besagte Verbindung der Formel (I) essentiell in ihrer R-isomerischen Form, ihrer S-isomerischen Form oder in ihrer razemischen Form vorliegt.

**20.** Die Verwendung nach einem der Ansprüche 17 - 18, wobei die Verbindung der Formel (I) (-)-(R)-1-(3-Hydroxypropyl)-5-[2-[[2-[2-(2,2,2-Trifluoroethoxy)Phenoxy]Ethyl]-Amino]Propyl]-Indolin-7- Carboxamid (KMD 3213) ist.

**21.** Die Verwendung nach einem der Ansprüche 17 - 20, wobei besagte Verbindung der Formel (I) in einer gleichmäßigen Fluxrate von mindestens 0,5 mg pro Tag durch Säugetierhaut verabreicht wird.

**22.** Die Verwendung nach einem der Ansprüche 17 - 21, wobei das Medikament zur transdermalen Anwendung die Vorrichtung zur transdermalen Verabreichung nach einem der Ansprüche 1 - 16 ist.

**23.** Die Verwendung nach einem der Ansprüche 17 - 21, wobei das Medikament zur transdermalen Anwendung eine Salbe, Creme, Spray, Gel oder Film ist.

## Revendications

**1.** Dispositif pour une administration transdermique, contenant un composé de formule (I)

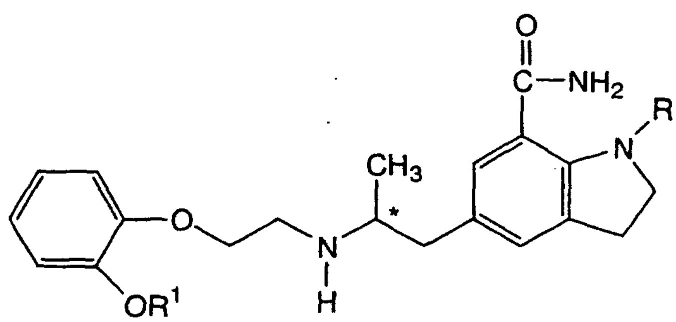

(I)

dans laquelle

R représente un groupe acyle aliphatique en $C_2$-$C_7$, saturé ou insaturé, éventuellement substitué par un ou plusieurs atomes d'halogènes, un groupe hydroxy, un groupe alcoxy en $C_1$-$C_6$, un groupe carboxyle, un groupe alcoxycarbonyle en $C_2$-$C_7$, un groupe cycloalkyle comportant de 5 à 7 chaînons, un groupe phényle ou naphtyle ; un groupe hydroxyalkyle en $C_2$-$C_6$ ; un groupe acyloxyalkyl aliphatique ayant un groupe acyle en $C_2$-$C_7$ et un groupe alkyle en $C_1$-$C_6$ ; un groupe alkyle en $C_1$-$C_6$ substitué par un groupe alcoxy en $C_1$-$C_6$, un groupe carboxyle, un groupe alcoxycarbonyle en $C_2$-$C_7$, un groupe alcoxycarbonyle en $C_2$-$C_7$ substitué par un groupe phényle ou naphtyle, un groupe carbamoyle, un groupe carbamoyle mono- ou di-substitué par un alkyle en $C_1$-$C_6$, ou un groupe cyano ; un groupe benzoyle ou naphtoyle éventuellement substitué par un ou plusieurs atomes

d'halogènes ; un groupe furoyle ou un groupe pyridylcarbonyle ; et

$R^1$ représente un groupe alkyle en $C_1$-$C_6$ éventuellement substitué par un ou plusieurs atomes d'halogènes, un groupe phényle ou naphtyle ;

l'atome de carbone affecté de l'astérisque * représente un atome de carbone en configuration (R), en configuration (S) ou un mélange des deux.

**2.** Dispositif pour une administration transdermique selon la revendication 1, ***caractérisé en ce que*** ledit composé de formule (I) est essentiellement sous sa forme isomère R, sous sa forme isomère S, ou sous sa forme racémique.

**3.** Dispositif pour une administration transdermique selon l'une quelconque des revendications précédentes, ***caractérisé en ce que*** ledit composé de formule (I) est le (-)-(*R*)-1-(3-hydroxypropyl)-5-[2-[[2-[2-(2,2,2-trifluoroéthoxy) phénoxy]éthyl]amino]propyl]indoline-7-carboxamide (KMD 3213).

**4.** Dispositif selon l'une quelconque des revendications précédentes, ***caractérisé en ce qu'il*** administre un composé de formule (I) à travers la peau de mammifères selon un taux de flux à l'état stationnaire d'au moins 0,5 mg par jour.

**5.** Dispositif selon l'une quelconque des revendications précédentes, ***caractérisé en ce qu'il*** a une charge d'un composé représenté par la formule (I) comprise entre 0,1 et 2 mg/$cm^2$ environ.

**6.** Dispositif selon l'une quelconque des revendications précédentes, ***caractérisé en ce qu'il*** comprend au moins une couche dans laquelle un composé de formule (I) est dissous selon une concentration d'au moins 1 % (poids/poids).

**7.** Dispositif selon la revendication 6, dans lequel le composé de formule (I) est dissous selon une concentration comprise entre 3 % et 7 % (poids/poids).

**8.** Dispositif selon l'une quelconque des revendications précédentes, ***caractérisé en ce qu'il*** s'agit d'un timbre du type à réservoir ou à matrice.

**9.** Dispositif selon l'une quelconque des revendications précédentes, ***caractérisé en ce qu'il*** s'agit d'un timbre du type à matrice, dans lequel ledit composé de formule (I) est dissous dans l'adhésif.

**10.** Dispositif selon l'une quelconque des revendications précédentes, ***caractérisé en ce qu'il*** contient un adhésif du type polyacrylate.

**11.** Dispositif selon l'une quelconque des revendications précédentes, ***caractérisé en ce qu'il*** comprend en plus un solubilisant.

**12.** Dispositif selon la revendication 11, dans lequel le solubilisant est un acide carboxylique.

**13.** Dispositif selon les revendications 11 ou 12, dans lequel le solubilisant est l'acide oléique.

**14.** Dispositif selon l'une quelconque des revendications 11 à 13, dans lequel le solubilisant est présent selon une proportion de 50 à 500 % molaires sur la base de la quantité du composé de formule (I) qui est incorporée dans le dispositif.

**15.** Dispositif selon l'une quelconque des revendications précédentes, dans lequel le dispositif possède une surface basale de 5 à 50 $cm^2$.

**16.** Dispositif selon l'une quelconque des revendications précédentes, ***caractérisé en ce qu'il*** libère le composé représenté par la formule (I) sur une période de temps prédéfinie, de manière préférée pendant 48 ou 72 heures, ou allant jusqu'à 7 jours.

**17.** Utilisation d'un composé de formule (I) :

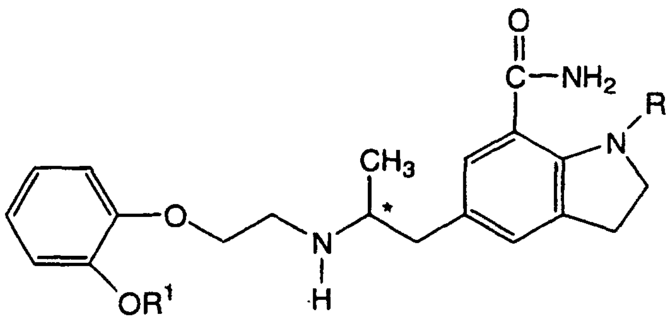

(I)

dans laquelle

R représente un groupe acyle aliphatique en $C_2$-$C_7$, saturé ou insaturé, éventuellement substitué par un ou plusieurs atomes d'halogènes, un groupe hydroxy, un groupe alcoxy en $C_1$-$C_6$, un groupe carboxyle, un groupe alcoxycarbonyle en $C_2$-$C_7$, un groupe cycloalkyle comportant de 5 à 7 chaînons, un groupe phényle ou naphtyle ; un groupe hydroxyalkyle en $C_2$-$C_6$ ; un groupe acyloxyalkyl aliphatique ayant un groupe acyle en $C_2$-$C_7$ et un groupe alkyle en $C_1$-$C_6$ ; un groupe alkyle en $C_1$-$C_6$ substitué par un groupe alcoxy en $C_1$-$C_6$, un groupe carboxyle, un groupe alcoxycarbonyle en $C_2$-$C_7$, un groupe alcoxycarbonyle en $C_2$-$C_7$ substitué par un groupe phényle ou naphtyle, un groupe carbamoyle, un groupe carbamoyle mono- ou di-substitué par un alkyle en $C_1$-$C_6$, ou un groupe cyano ; un groupe benzoyle ou naphtoyle éventuellement substitué par un ou plusieurs atomes d'halogènes ; un groupe furoyle ou un groupe pyridylcarbonyle ; et

$R^1$ représente un groupe alkyle en $C_1$-$C_6$ éventuellement substitué par un ou plusieurs atomes d'halogènes, un groupe phényle ou naphtyle ;

l'atome de carbone affecté de l'astérisque * représente un atome de carbone en configuration (R), en configuration (S) ou un mélange des deux,

pour la préparation d'un médicament pour une application transdermique destiné au traitement ou à la prévention d'un trouble des voies urinaires et/ou des symptômes associés à ce trouble.

**18.** Utilisation selon la revendication 17, dans laquelle le trouble des voies urinaires est l'hypertrophie (bégnine) de la prostate.

**19.** Utilisation selon l'une quelconque des revendications 17 et 18, ***caractérisée en ce que*** ledit composé de formule (I) est essentiellement sous sa forme isomère R, sous sa forme isomère S, ou sous sa forme racémique.

**20.** Utilisation selon l'une quelconque des revendications 17 et 18, dans laquelle le composé de formule (I) est le (- ) -(*R*)-1-(3-hydroxypropyl)-5-[2-[[2-[2-(2,2,2-trifluoroéthoxy) phénoxy]éthyl]amino]propyl]indoline-7-carboxamide (KMD 3213).

**21.** Utilisation selon l'une quelconque des revendications 17 à 20, dans laquelle ledit composé de formule (I) est administré à travers la peau de mammifères selon un taux de flux à l'état stationnaire d'au moins 0,5 mg/jour.

**22.** Utilisation selon l'une quelconque des revendications 17 à 21, dans laquelle le médicament d'application transdermique est le dispositif pour administration transdermique selon l'une quelconque des revendications 1 à 16.

**23.** Utilisation selon l'une quelconque des revendications 17 à 21, dans laquelle le médicament d'application transdermique est un onguent, une crème, une pulvérisation, un gel ou un film.

Fig. 1

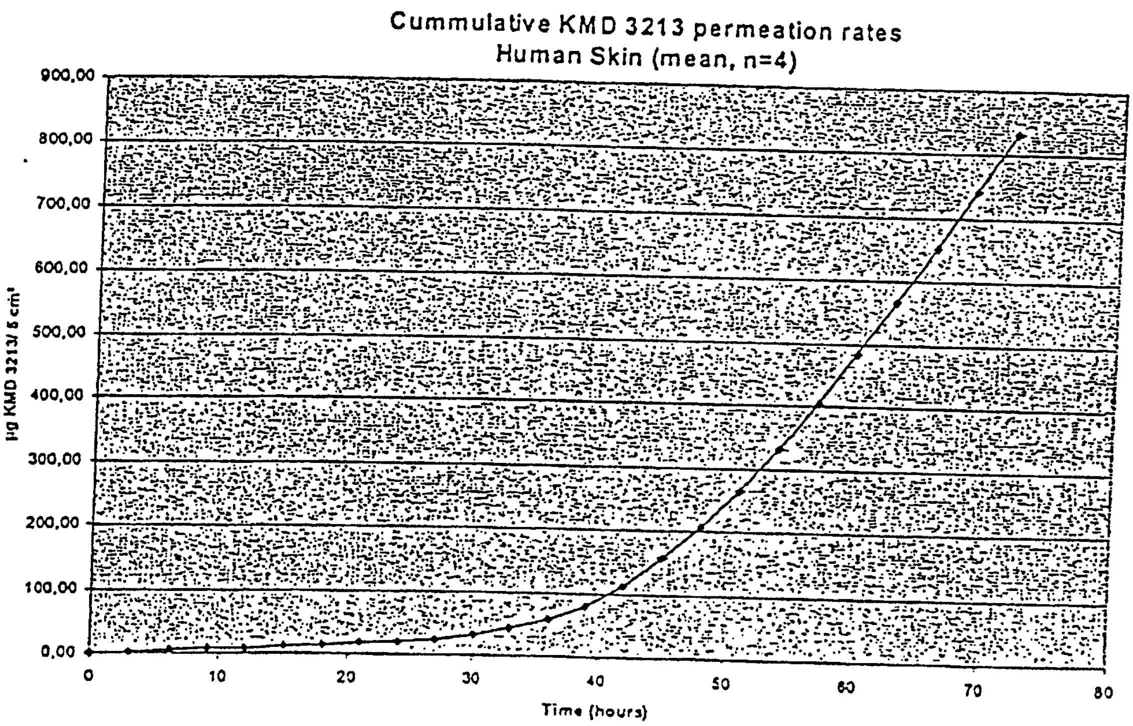
Cummulative KMD 3213 permeation rates
Human Skin (mean, n=4)
900,00
800,00
700,00
600,00
500,00
400,00
300,00
200,00
100,00
0,00
µg KMD 3213/ 5 cm²
0
10
20
30
40
50
60
70
80
Time (hours)